# EUROPÄISCHE PATENTSCHRIFT

(11) **EP 1 216 047 B1**
(45) Veröffentlichungstag und Bekanntmachung des Hinweises auf die Patenterteilung: **12.10.2005**
(21) Anmeldenummer: 00969304.5
(22) Anmeldetag: 26.09.2000
(51) Int. Cl.: A61K 31/57, A61K 31/165, A61K 31/56, A61P 11/00, A61P 11/06, A61K 31/167, A61K 9/12, A61K 31/522, A61K 31/135

(54) **KOMBINATION VON LOTEPREDNOL UND BETA2-ADRENOZEPTOR-AGONISTEN UND IHRE VERWENDUNG DURCH INHALATION ZUR BEHANDLUNG VON ASTHMA BRONCHIALE**
COMBINATION OF LOTEPREDNOL AND BETA2-ADRENOCEPTOR AGONISTS AND ITS USE BY INHALATION FOR THE TREATMENT OF BRONCHIAL ASTHMA
ASSOCIATION DE LOTEPREDNOL ET D'AGONISTES DE L'ADRENOCEPTEUR BETA2 ET SON UTILISATION PAR INHALATION POUR LE TRAITEMENT DE L'ASTHME BRONCHIQUE

(30) Priorität: 30.09.1999 DE 19947235
(43) Veröffentlichungstag der Anmeldung: 26.06.2002
(73) Patentinhaber: VIATRIS GmbH & Co. KG, 61352 Bad Homburg (DE)
(72) Erfinder: SZELENYI, Istvan, 90571 Schwaig (DE); POPPE, Hildegard, 01109 Dresden (DE); HEER, Sabine, 01445 Radebeul (DE); ENGEL, Jürgen, 63755 Alzenau (DE)
(86) Internationale Anmeldenummer: PCT/EP2000/009392
(87) Internationale Veröffentlichungsnummer: WO 2001/022956

(56) Entgegenhaltungen:
- WO-A-00/28979
- US-A- 5 830 490
- L.BJERMER, L.LARSSON: "Long-acting beta-2-agonists: how are they used in an optimal way?" RESPIRATORY MEDICINE, Bd. 91, Nr. 10, 1997, Seiten 587-591, XP000992766
- T. VAN DER MOLEN E.A.: "Effects of the long acting beta agonist formoterol on asthma control in asthmatic patients using inhaled corticosteroids" THORAX, Bd. 52, Nr. 6, 1997, Seiten 535-539, XP000992767

## Beschreibung

Die Erfindung betrifft eine neue Kombination von dem Soft-Steroid Loteprednol und mindestens einem β2-Adrenozeptor-Agonisten zur simultanen, sequentiellen oder separaten Behandlung durch Inhalation von Asthma bronchiale , die Kombination enthaltende Arzneimittel, Verfahren zur Herstellung solcher Arzneimittel und die Verwendung der neuen Kombination zur Herstellung von Arzneimitteln zur simultanen, sequentiellen oder separaten Behandlung durch Inhalation von Asthma bronchiale.

### Hintergrund der Erfindung

Weltweit nimmt die Zahl der Patienten, die an Allergien und/oder Atemwegserkrankungen, wie Asthma bronchiale, leiden, stark zu.

Studien haben ergeben, daß in den industrialisierten Ländem, 5 - 10 % der Bevölkerung an Asthma leiden. Trotz intensiver Forschungsaktivität ist die Pathogenese des bronchialen Asthma immer noch nicht vollständig geklärt. Obwohl in den vergangenen Jahren zahlreiche neue effektive Arzneimittel in der Therapie eingeführt worden sind, ist die Behandlung dieser Erkrankung immer noch nicht zufriedenstellend. Besonders besorgniserregend ist die Tatsache, daß viele Asthmatiker nicht ausreichend therapiert worden.

Asthma bronchiale ist eine Erkrankung der unteren Atemwege. Sie äußert sich in der Kontraktion der bronchialen glatten Muskulatur, die zu einer akuten Atemnot führt. Neben dieser Bronchokonstriktion steht aber eine chronische Entzündung im Vordergrund des asthmatischen Geschehens. Diese chronische, stets fortschreitende Entzündung kann unter Umständen zur weiteren Schädigung der bronchialen Schleimhaut und somit zu strukturellen Veränderungen des Bronchialbaumes führen. Infolge dieser Schädigungen kann eine irreversible Einengung der Bronchien auftreten. Demzufolge ist es ertorderlich, die Asthmatiker so zu behandeln, daß sie frei von den akuten asthmatischen Anfällen sind und gleichzeitig die der Erkrankung zugrunde liegende Entzündung reduziert wird. Um einen akuten Asthmaanfall zu beheben bzw. seinem Auftreten vorzubeugen, eignen sich die β2-Adrenozeptor-Agonisten am besten. Inzwischen sind β2-Agonisten mit Kurz- und Langzeitwirkung auf dem Markt, Um β2-Adrenozeptor-bedingte Nebenwirkungen, die unter Umständen lebensbedrohlich sein können, zu vernneiden, sollten die lang anhaltend wirksamen β2-Adrenozeptor-Agonisten nur zweimal am Tag angewendet werden. Demgegenüber kann von den sogenannten Kurzzeit- β2-Adrenozeptor-Agonisten bei Bedarf Gebrauch gemacht werden. Asthmatiker setzen β2-Adrenozeptor-Agonisten gem ein, weil sie die akuten Symptome sofort beheben. Weniger bewußt werden die antientzündlichen Medikamente, wie Corticosteroide angewandt, da sie die Atemnot nicht beseitigen und somit der Patient keine unmittelbare Verbesserung seines Zustandes verspürt. Allerdings ist die Suppiementation der inhalativen Therapie von Asthma bronchiale mit "klassischen" Corticosteroiden durch Langzeit-β2-Adrenozeptor-Agonisten aus dem Stand der Technik bekannt (Respiratory Medicine, 1997; 91, S. 587-591).

Loteprednol gehört zu den sogenannten "soft" Corticosteroiden. Diese sogenannten "soft" Corticosteroide (Softsteroide) zeichnen sich dadurch aus, daß sie durch eine sogenannte Ein-Schritt-Reaktion z.H. durch Hydrolasen, Esterasen ohne Beteiligung der überwiegend hepatisch lokalisierten Cytochrom P450 Monooxydase-Enzyme inaktiviert werden. Dadurch kommt es, wenn überhaupt, nur zu sehr geringen Plasmakonzentrationen, die nicht ausreichen, um die klassischen Corticosteroid-Nebenwirkungen wie Wachstumsretardierung, Osteoporose, Erhöhung des intraokulären Druckes hervorzurufen.

Überraschenderweise wurde nun gefunden, daß die neue Kombination des Soft-Steroids Loteprednol mit mindestens einem β2-Adrenozeptor-Agonisten bei der Behandlung durch Inhalation von Asthma bronchiale bei Säugetieren, insbesondere beim Menschen, vorteilhaft ist. Die Verabreichung der Kombination kann simultan, sequentiell oder separat zur topischen Behandlung durch Inhalation erfolgen.

In der Ausführungsform der Erfindung wird Loteprednol beziehungsweise dessen pharmazeutisch annehmbare Ester, insbesondere Loteprednol Etabonat als Soft-Steroid eingesetzt. Die Herstellung von Loteprednol und Loteprednol Etabonat ist beispielsweise in dem deutschen Patent Nr. DE 31 26 732, dem korrespondierenden US-Patent Nr. 4,996,335 und dem korrespondierenden japanischen Patent Nr. JP-89 011 037 beschrieben.

Loteprednol ist zur Behandlung der allergischen Conjunctivitis und Uveitis in den Vereinigten Staaten zugelassen. Dabei wurde es gezeigt, daß topisch verabreichtes Loteprednol den intraokulären Druck im Gegensatz zu den nicht-"soft" Corticosteroiden nicht erhöht und im Plasma war es nicht nachzuweisen (Noble and Goa, BioDrugs 10:329-339, 1998). Infolge der genannten Ein-Schritt-Reaktion wird bei der inhalativen Gabe auch der verschluckte Anteil in der Leber sofort inaktiviert. Daher kann auch dieser Anteil keine Nebenwirkungen hervorrufen.

β2-Adrenozeptor-Agonisten sind Medikamente, die die β2-Adrenorezeptoren selektiv stimulieren und dadurch die Bronchien zur Relaxation bringen. Außerdem hemmen sie über die Hemmung der Freisetzung mancher endogener Mediatoren auch die Ödembildung und fördem die mukoziliäre Clearence. Sie beheben der akuten Anfall (Atemnot infolge der Bronchokonstriktion) sehr schnell. Ihre Wirkung hält unterschiedlich lange an: die z.B. von Salbutamol (Kurzzeit- β2-Adrenozeptor-Agonisten) 4-6 Stunden, die von Formoterol oder Salmeterol (Langzeit- β2-Adrenozeptor-Agonisten) bis zu 12 Stunden. Ein großer Vorteil von Formoterol dem Salmeterol gegenüber ist, daß die bronchodilatatorische Wirkung von Formoterol nicht nur lange anhält sondem sofort wie bei den Kurzzeit β2-Adrenozeptor-Agonisten eintritt (Palmqvist et al., J. Allergy Clin. Immunol. 89:844-849, 1992).

Formoterol, Salmeterol und Salbutamol können auch in Form der pharmazeutisch verträglichen Salze verwendet werden, wobei Formoterol-Fumarat, Salmeterolxinafoat und Salbutamolsulfat bevorzugt sind. Besonders bevorzugt ist Formoterol-Fumarat-Dihydrat.

Gemäß einer besonderen Ausführungsform kann als β2-Adrenozeptor-Agonist Reproterol oder dessen pharmazeutisch verträglichen Salze verwendet werden, wobei Reproterolhydrochlorid bevorzugt ist.

Die Verabreichung dieser Wirkstoffe erfolgt als Inhalation mit Hilfe von Dosieraerosolen (MDI) oder Pulverinhatatoren (MPDI). Durch die inhalative Verabreichung kann nicht nur die Dosis sondem auch das Auftreten möglicher unerwünschter Wirkungen reduziert werden.

Die vorliegende Erfindung beschreibt die Kombination des "soft" Corticosteroid Loteprednol mit einem β2-Adrenozeptor-Agonisten, vorzugsweise Salbutamol oder Formoterol, wobei die Einzelkomponente dieser Kombination in der Therapie des Asthma bronchiale gleichzeitig, hintereinander oder einzeln inhalativ verabreicht werden können. Besonders vorteilhaft ist eine fixe Kombination der beiden wirksamen Komponenten, da in diesem Fall der Patient nur ein Dosieraerosol braucht und somit die wirksame Behandlung für den Patienten leichter ist.

Die Gründe für die in der Erfindung beschriebene Kombination lassen sich experimentell untermauem.

*In vitro* wurden Untersuchungen zur Beeinflussung der Freisetzung des proinflammatorischen Cytokins TNFα im 1:5 verdünnten Humanblut durchgeführt. Die Stimulation erfolgte mit Lipopolysaccharid (LPS) von Salmonella abortus equi (10 µg/ml) über 24 h bei 37°C und 5% CO₂ im Brutschrank. Die Bestimmung der TNFα-Freisetzung erfolgte mit einem ELISA, aufgebaut aus Antikörpem der Fa. Pharmigen. Die Ergebnisse wurden als prozentuale Hemmung der LPS-induzierten TNFα-Freisetzung angegeben (Tabelle 1).

**Tabelle 1:**

| Hemmung der TNFα-Freisetzung im 1:5 verdünnten Humanblut (n=8) | | |
|---|---|---|
| Wirkstoff | Konzentration [µmol/l] | Hemmung der TNFα-Freisetzung |
| Salbutamol | 10 | 17% |
| Loteprednol | 0,001 | 1 % |
| Loteprednol + Salbutamol | 0,001+10 | 44* % |

| | | |
|---|---|---|
| * (P<0,01) | | |

*In vivo* Untersuchungen wurden an Meerschweinchen durchgeführt, die durch zweimalige intraperitoneale (i.p.) Injektion einer Suspension aus Ovalbumin und Aluminiumhydroxid in physiologischer Kochsalzlösung an zwei aufeinander folgenden Tagen aktiv sensibilisiert wurden. Zwei Wochen nach der Sensibilisierung wurden die in einer Vemebelungsbox kurzzeitig einem Aerosol aus Ovalbuminlösung ausgesetzt. Durch die inhalative Allergen-Provokation kommt es 24 Stunden später zu einem starken Anstieg der Anzahl eosinophiler Granulozyten (Entzündungszellen) in der Lunge. Ähnlich wie bei den Asthmatikem erfolgt zu diesem Zeitpunkt (24 Stunden nach der allergischen Provokation) eine Spülung der Lunge. Die Anzahl der eosinophilen Granulozyten in der Lungenspülflüssigkeit wird mit einem Hämazytometer (Technicon H1E) bestimmt. Anschließend wird die prozentuale Hemmung der eosinophilen Granulozyten durch Testsubstanzen berechnet.

Um die Wirkstoffe genau dosiert intrapulmonal geben zu können, werden sie den Tieren durch einen in die Trachea eingebundenen Katheter als Pulver (mit Laktose vermischt) direkt in die Lunge verabreicht. Die Applikation der Wirkstoffe erfolgt vor der Allergen-Provokation in kurzer Ketamin/Xylazin-Narkose, aus der die Tiere sofort aufwachen. Die Ergebnisse sind in der Tabelle 2 zusammengestellt.

**Tabelle 2:**

| Die Wirkung von Loteprednol und Formoterol alleine und in Kombination auf die Spätphasen-Eosinophilie in aktiv sensibilisierten Meerschweinchen | | | |
|---|---|---|---|
| Wirkstoff | Dosis in mg/kg intrapulmonal | Hemmung der Eosinophilie | Anzahl der Tiere |
| Loteprednol | 0,001 | 10,5% | 5 |
| | 0,003 | 21,8% | 5 |
| Formoterol | 0,0001 | 4,1% | 4 |
| | 0,001 | 20,4% | 4 |
| Loteprednol + Formoterol | 0,001 + 0,0001 | 36,1 %* | 6 |
| | 0,003 + 0,0001 | 45,21 %* | 6 |
| | 0,001 + 0,001 | 64,5 %** | 6 |

| | | | |
|---|---|---|---|
| signifikant gegenüber Allergen-Provokation-Kontrolle: * (0,05); | | | |
| ** (p<0.01) | | | |

Wenn das "soft" Corticosteroid Loteprednol in der Dosis 0,001 mg/kg oder der β2-Agonist Formoterol in den Dosierungen 0,0001 mg/kg und 0,001 mg/kg bei intrapulmonaler Pulverapplikation untersucht wurde, kam es zu keiner oder einer marginalen Hemmung der allergisch induzierten Spätphasen-Eosinophilie. Wenn beide Wirkstoffe gleichzeitig gegeben wurden, konnte die Anzahl der eosinophilen Granulozyten in der Lungenspülflüssigkeit 24 Stunden nach Allergen-Provokation um 39,1 % bzw. 64,5 % (signifikant) reduziert werden.

Wie bereits kurz erwähnt wurde, rufen die Corticosteroide zahlreiche Nebenwirkung hervor, die ihre klinische Anwendung oft einschränkt. Besonders bei Kindern beeinflussen Corticosteroide das Wachstum. Im allgemeinen kann man sagen, daß das Wachstum der mit Corticosteroiden behandelten asthmatischen Kinder jährlich um einen Zentimeter (1 cm) hinter den nicht mit Corticosteroiden behandelten zurückbleibt. Diese unerwünschte Nebenwirkung gilt für alle zum gegenwärtigen Zeitpunkt auf dem Markt befindlichen Corticosteroide wie z.B. Budesonid oder Fluticason (vgl. Clissold S.P. and R.C. Heel., Drugs 28, 485-518, 1984; Shaw R.J., Respiratory Medicine 88(Suppl. A):5-8, 1994; Bames P.J. *et al.,* Am. J. Resp. Critical Care Med. 157(3)Suppl. Part 2:S1-S53, 1998;). Bei einem Corticosteroid könnte es von großem Vorteil sein, wenn dieses Corticosteroid die Wachstumsentwicklung bei Kindern nicht beeinflussen würde. Um das Nebenwirkungspotential tierexperimentell zu erfassen, untersucht man den Einfluß der Corticosteroide auf die Thymusdrüse der Ratte.

Im ersten Versuch wurde Loteprednol im Vergleich zu Fluticason, Bectomethason und Budesonid ausgewachsenen Ratten über 5 Tage 1 x täglich subcutan appliziert. Bis zur Dosis 10 mg/kg s.c. Loteprednol konnte keine signifikante Reduktion des Thymusgewichtes gegenüber Kontrolltieren gemessen werden. Fluticason (1 mg/kg s.c.), Beclomethason (1 mg/kg s.c.) und Budesonid (2 mg/kg s.c.) zeigten eine signifikante Reduktion der Thymusgewichte (siehe Tabelle 3).

**Tabelle 3:**

| Wirkung von Corticosteroiden in hohen Dosen auf das Thymusgewicht von Ratten bei wiederholter subcutaner Applikation (5 Tage, je 1 x täglich) | | |
|---|---|---|
| Wirkstoff | Dosis in mg/kg subcutane Gabe Mehrfachgabe (5 Tage, je 1 x täglich) | % Reduktion des Thymusgewichtes (mg/100 g KM) gegenüber Laktose-Kontrolle 0 |
| Loteprednol | 1 | 15 |
| | 10 | 28 |
| Fluticason | 1 | 65 (p<0,01) |
| Beclomethason | 1 | 51 (p<0,01) |
| Budesonid | 2 | 89 (p<0,05) |

Im zweiten Versuch wurde der Einfluß von Loteprednol auf die Thymusentwicklung von jungen Ratten (bei Versuchsbeginn 21 Tage alt) im Vergleich zu Budesonid und Fluticason intensiv untersucht (siehe Tabelle 4). Nach einer intrapulmonalen Langzeitgabe des Wirkstoffes in Pulverform über 29 Tage (1 x täglich) mittels Sonde wurden am Versuchsende die Thyrnusdrüsen entnommen und das Organgewicht pro 100 g Körpermasse bestimmt. Fluticason bewirkte in der Dosis 1,0 mg/kg und Budesonid in der Dosis 0,5 mg/kg eine signifikante Reduktion des Thymusgewichtes gegenüber Kontrolltieren, die mit Laktose behandelt wurden, Loteprednol zeigte bei intrapulmonaler Langzeitgabe erst in der hohen Dosis 20 mg/kg eine deutliche Reduktion des Thymusgewichtes.

Die therapeutische Breite der Cortikosteroide wurde mit Hilfe des Quotienten aus der Dosis (mg/kg) mit signifikanter Thymusinvolution (toxische Dosis) bei wiederholter intrapulmonaler Gabe über 29 Tage (1 x täglich) und der therapeutischen Dosis bestimmt. Die therapeutische Dosis wurde am Asthma-Modell der Spätphasen-Eosinophilie an aktiv sensibisierten Brown Norway Ratten ermittelt. Der Wirkstoff wurde den sensibilisierten Brown Norway Ratten in Narkose einmalig intrapulmonal 2 Stunden vor einer Allergen-Provokation (Challenge) verabreicht und 24 Stunden später die Lungen der Tiere in tiefer Narkose gespült. Anschließend wurde die Anzahl der eosinophilen Granulozyten in der Lungenspülflüssigkeit von unbehandelten und mit Wirkstoff behandelten Ratten bestimmt. Corticosteroide wie Loteprednol hemmen die Einwanderung von Eosinophilen in die Lunge nach Allergen-Provokation. Aus den Hemmwerten steigender Wirkstoffdosen wurde die 50%ige inhibitorische Dosis (ID₅₀ in µg/kg) an der allergisch induzierten Spätphasen-Eosinophilie ermittelt.

Für Loteprednol konnte eine große therapeutische Breite mit einem Quotienten von 45,5 x 10³ ermittelt werden. Budesonid (5 x 10³) und Fluticason (33 x 10³) wiesen deutlich kleinere Quotienten auf (siehe Tabelle 4).

**Tabelle 4:**

| Therapeutische Breite von Corticosteroiden an Ratten bei intrapulmonaler Pulverapplikation Quotient aus toxischer Dosis (mg/kg) mit signifikanter Thymusinvolution bei wiederholter Gabe (29 Tage) und therapeutischer Dosis ((ID₅₀ µg/kg) bei Einmalgabe an der Spätphasen-Eosinophilie in aktiv sensibilisierten Brown Norway Ratten. | | | |
|---|---|---|---|
| Wirkstoff | therapeutische Dosis an der Spätphasen-Eosinophille ID₅₀ in µg/kg | toxische Dosis mit signifikanter Thymusinvolution in mg/kg | Quotient aus toxischer Dosis: therapeutischer Dosis |
| Dosierung | Einmalgabe, 2 h vor Allergen-Challenge | wiederholte Gabe, 29 Tage, 1 x täglich | x 10³ |
| Applikation | intrapulmonal | intrapulmonal | |
| | | | |
| Loteprednol | 0.44 | 20 | 45.5 |
| Fluticason | 0.03 | 1 | 33 |
| Budesonid | 0.1 | 0.5 | 5 |
| | | | |

Damit ist Loteprednol hinsichtlich der therapeutischen Breite den Steroiden Fluticason und Budesonid deutlich überlegen.

Die Menge an zu verabreichendem Loteprednol und β2-Adrenorezeptor Agonist hängt unter anderem von der Wirksamkeit, Wirkungsdauer und der Art und dem Schweregrad der zu behandelnden Krankheit ab. Weiterhin ist die Konstitution und das Alter des Patienten von Bedeutung. Das Verhältnis Formoterol zu Loteprednol kann beispielsweise im Bereich zwischen 2:1 und 1:500, vorzugsweise zwischen 1:8 und 1:63, besonders bevorzugt zwischen 1.8 und 1:42, jeweils bezogen auf das Gewicht, liegen. So hat sich beispielsweise ein Verhältnis von 1:10 bis 1:35 als günstig erwiesen. Die beiden Komponenten können simultan, sequentiell oder separat in einer pharmazeutischen Zubereitung, die die Komponenten getrennt oder zusammen, gegebenenfalls mit den üblichen Hilfs- oder Trägerstoffen, enthält verabreicht werden.

Wegen der langen Wirkdauer beider Wirkstoffe ist eine täglich zweimalige Dosierung bevorzugt. Ein angemessener Dosisbereich von Formoterol liegt zwischen 6 und 100 µg/die, wobei ein Dosisbereich von 6 bis 48 µg/die bevorzugt ist. Für Loteprednol kann man den täglichen angemessenen Dosisbereich mit 50 bis 2000 µg/die angeben. Bevorzugt wird eine tägliche Dosierung von 100 bis 1000 µg/die. Wegen der im Tierexperiment und auch in der Behandlung der allergischen Conjunctivitis nachgewiesenen Unbedenklichkeit kann Loteprednol in der Kombination täglich auch bis auf 3000 µg dosiert werden.

### Beispiel 1:

### Dosieraerosol mit 6 µg Formoterol Fumarat Dihydrat und 200 µg Loteprednol Etabonat pro Hub

1,000 g 2H-Heptafluorpropan (= Treibmittel 227) werden auf eine Temperatur von etwa -55 °C abgekühlt und unter Rühren mit einer Lösung aus 11,7 g Polyoxyethylen-25-glyceryl-trioleat (Handelsname: Tagat®T0, Goldschmidt AG) in 11,7 g absolutem Ethanol versetzt. Anschließend werden 3,34 g mikronisiertes Loteprednol Etabonat und 0,1 g mikronisiertes Formoterol Fumarat Dihydrat zugesetzt und die entstehende Suspension intensiv homogenisiert. Unter weiterem Rühren und Kühlen wird die Suspension mit gekühltem Treibmittel 227 auf 1.170,0 g aufgefüllt und sodann in Metalldosen abgefüllt, die mit Dosierventilen verschlossen werden, welche pro Hub 50 µl der Suspension freisetzen.

Pro Hub werden 6 µg Formoterol Fumarat Dihydrat und 200 µg Loteprednol Etabonat freigesetzt.

### Beispiele 2 bis 4:

Es wird gearbeitet wie im Beispiel 1, jedoch werden statt der dort genannten Mengen an Wirksubstanzen folgende Mengen eingesetzt:

| | **Pro Ansatz eingesetzter Wirkstoff** | | **Pro Hub freigesetzter Wirkstoff** | |
|---|---|---|---|---|
| **Beispiel** | **Formoterol Fumarat Dihydrat** | **Loteprednol Etabonat** | **Formoterol Fumarat Dihydrat** | **Loteprednol Etabonat** |
| 2 | 0,2 g | 3,34 g | 12 µg | 200 µg |
| 3 | 0,2 g | 8,35 g | 12 µg | 500 µg |
| 4 | 0,4 g | 8,35 g | 24 µg | 500 µg |

### Beispiel 5:

### Pulverinhalation mit 6 µg Formoterol Fumarat Dihydrat und 200 µg Loteprednol Etabonat pro Einzeldosis

0,51 g mikronisiertes Formoterol Fumarat Dihydrat worden mit 10 g α-Lactose Monohydrat gemischt, die Mischung durch ein Sieb der Maschenweite 0,3 mm gesiebt und im Turbula-Mischer (Hersteller: Bachofen, Basel, Schweiz) 10 Minuten lang gemischt.

17 g Loteprednol Etabonat werden mit 340 g α-Lactose Monohydrat gemischt, die Mischung durch ein Sieb der Maschenweite 0,3 mm gesiebt und im Turbula-Mischer 10 Minuten lang gemischt.

Die beiden Mischungen werden vereinigt, die Mischung emeut durch ein Sieb der Maschenweite 0,3 mm gesiebt, mit α-Lactose Monohydrat auf 1.020 g aufgefüllt und nochmals 30 Minuten lang im Turbula-Mischer gemischt.

Die Mischung wird in einen Pulverinhalator gefüllt, der pro Einzeldosis 12 µg der Mischung freisetzt. 12 mg der Mischung enthalten 6 µg Formoterol Fumarat Dihydrat und 200 µg Loteprednol Etabonat.

### Beispiele 6-8:

Es wird gearbeitet wie in Beispiel 5, jedoch werden statt der dort genannten Mengen an Wirksubstanzen folgende Mengen eingesetzt:

| | **Pro Ansatz eingesetzter Wirkstoff** | | **Pro Einzeldosis enthaltener Wirkstoff** | |
|---|---|---|---|---|
| **Beispiel** | **Formoterol Fumarat Dihydrat** | **Loteprednol Etabonat** | **Formoterol Fumarat Dihydrat** | **Loteprednol Etabonat** |
| 6 | 1,02 g | 17,0 g | 12 µg | 200 µg |
| 7 | 1,02 g | 42,5g | 12 µg | 500 µg |
| 8 | 2,04 g | 42,5 g | 24 µg | 500 µg |

## Patentansprüche

1. Pharmazeutische Zubereitung enthaltend, getrennt oder zusammen, eine wirksame Menge an (i) Loteprednol oder einen pharmazeutisch verträglichen Ester davon und (ii) mindestens einem β2-Adrenozeptor-Agonisten in einer Form geeignet für die simultane, sequentielle oder separate Verabreichung durch Inhalation bei der Behandlung von Asthma bronchiale bei Säugetieren.

2. Pharmazeutische Zubereitung nach Anspruch 1, **dadurch gekennzeichnet, daß** es sich bei dem pharmazeutisch verträglichen Ester des Loteprednol um Loteprednol Etabonat handelt.

3. Pharmazeutische Zubereitung nach Anspruch 1 oder 2, **dadurch gekennzeichnet, daß** der β2-Adrenozeptor-Agonist aus der Gruppe bestehend aus Salbutamol, Reproterol, Salmeterol, Formoterol und deren pharmazeutisch verträglichen Salze ausgewählt ist.

4. Pharmazeutische Zubereitung nach einem der Ansprüche 1 bis 3, **dadurch gekennzeichnet, daß** sie (i) Loteprednol und (ii) Formoterol enthält.

5. Pharmazeutische Zubereitung nach einem der Ansprüche 1 bis 3, **dadurch gekennzeichnet, daß** sie (i) Loteprednol und (ii) Salmeterol enthält.

6. Pharmazeutische Zubereitung nach einem der Ansprüche 1 bis 3, **dadurch gekennzeichnet, daß** sie (i) Loteprednol und (ii) Reproterol enthält.

7. Arzneimittel enthaltend eine wirksame Menge an (i) Loteprednol und (ii) mindestens einen β2-Adrenorezeptor-Agonisten, gegebenenfalls zusammen mit üblichen Hilfs- oder Trägerstoffen in einer Form geeignet zur simultanen, sequentiellen oder separaten Verabreichung durch Inhalation bei der Behandlung von Asthma bronchiale.

8. Verfahren zur Herstellung eines Arzneimittel in einer Form geeignet zur inhalativen Behandlung von Asthma bronchiale, enthaltend den Wirkstoff Loteprednol und mindestens einen β2-Adrenorezeptor-Agonisten, **dadurch gekennzeichnet, daß** man Loteprednol und den β2- Adrenorezeptor-Agonisten oder die β2-Adrenorezeptor-Agonisten, einzeln oder zusammen, gegebenenfalls zusammen mit üblichen Hilfs- oder Trägerstoffen, vermischt und die so erhaltene Mischung in geeignete Darreichungsformen überführt.

9. Verwendung der Kombination aus Loteprednol und mindestens einem β2-Adrenorezeptor-Agonisten zur Herstellung eines Arzneimittels zur simultanen, sequentiellen oder separaten inhalativen Behandlung von Asthma bronchiale.

## Claims

1. Pharmaceutical preparation comprising, separately or together, an efficacious amount of (i) loteprednol or a pharmaceutically tolerable ester thereof and (ii) at least one β2 adrenoceptor agonist in a form which is suitable for simultaneous, sequential or separate administration by inhalation in the treatment of bronchial asthma in mammals.

2. Pharmaceutical preparation according to Claim 1, **characterized in that** the pharmaceutically tolerable ester of loteprednol is loteprednol etabonate.

3. Pharmaceutical preparation according to Claim 1 or 2, **characterized in that** the β2 adrenoceptor agonist is selected from the group consisting of salbutamol, reproterol, salmeterol, formoterol and their pharmaceutically tolerable salts.

4. Pharmaceutical preparation according to one of Claims 1 to 3, **characterized in that** it contains (i) loteprednol and (ii) formoterol.

5. Pharmaceutical preparation according to one of Claims 1 to 3, **characterized in that** it contains (i) loteprednol and (ii) salmeterol.

6. Pharmaceutical preparation according to one of Claims 1 to 3, **characterized in that** it contains (i) loteprednol and (ii) reproterol.

7. Medicament comprising an efficacious amount of (i) loteprednol and (ii) at least one β2 adrenoreceptor agonist, if appropriate together with customary excipients or vehicles, in a form which is suitable for simultaneous, sequential or separate administration by inhalation in the treatment of bronchial asthma.

8. Process for the production of a medicament in a form which is suitable for the inhalative treatment of bronchial asthma, comprising the active compound loteprednol and at least one β2 adrenoreceptor agonist, **characterized in that** loteprednol and the β2 adrenoreceptor agonist or the β2 adrenoreceptor agonists are mixed individually or together, if appropriate together with customary excipients or vehicles, and the mixture thus obtained is converted into suitable administration forms.

9. Use of the combination of loteprednol and at least one β2 adrenoreceptor agonist for the production of a medicament for the simultaneous, sequential or separate inhalative treatment of bronchial asthma.

## Revendications

1. Préparation pharmaceutique contenant, séparément ou ensemble, une quantité efficace de (i) lotéprednol ou d'un ester pharmaceutiquement acceptable de celui-ci et (ii) d'au moins un agoniste de récepteur adrénergique β2 sous une forme appropriée à l'administration par inhalation, simultanée, séquentielle ou séparée, dans le traitement de l'asthme bronchique chez des mammifères.

2. Préparation pharmaceutique selon la revendication 1, **caractérisée en ce que** l'ester pharmaceutiquement acceptable du lotéprednol consiste en lotéprednol étabonate.

3. Préparation pharmaceutique selon la revendication 1 ou 2, **caractérisée en ce que** l'agoniste de récepteur adrénergique β2 est choisi dans le groupe constitué par le salbutamol, le réprotérol, le salmétérol, le formotérol et leurs sels pharmaceutiquement acceptables.

4. Préparation pharmaceutique selon l'une quelconque des revendications 1 à 3, **caractérisée en ce qu'**elle contient (i) du lotéprednol et (ii) du formotérol.

5. Préparation pharmaceutique selon l'une quelconque des revendications 1 à 3, **caractérisée en ce qu'**elle contient (i) du lotéprednol et (ii) du salmétérol.

6. Préparation pharmaceutique selon l'une quelconque des revendications 1 à 3, **caractérisée en ce qu'**elle contient (i) du lotéprednol et (ii) du réprotérol.

7. Médicament contenant une quantité efficace de (i) lotéprednol et (ii) d'au moins un agoniste de récepteur adrénergique β2, éventuellement conjointement avec des adjuvants ou véhicules usuels, sous une forme appropriée à l'administration par inhalation, simultanée, séquentielle ou séparée, dans le traitement de l'asthme bronchique.

8. Procédé pour la fabrication d'un médicament sous une forme appropriée au traitement par inhalation de l'asthme bronchique, contenant la substance active lotéprednol et au moins un agoniste de récepteur adrénergique β2, **caractérisé en ce qu'**on mélange le lotéprednol et l'agoniste de récepteur adrénergique β2 ou les agonistes de récepteur adrénergique β2, seuls ou ensemble, éventuellement conjointement avec des adjuvants ou véhicules usuels, et on met sous une forme d'administration appropriée le mélange ainsi obtenu.

9. Utilisation de l'association de lotéprednol et d'au moins un agoniste de récepteur adrénergique β2 pour la fabrication d'un médicament destiné au traitement par inhalation, simultané, séquentiel ou séparé, de l'asthme bronchique.
